# EUROPEAN PATENT APPLICATION

(11) **EP 2 275 529 A2**
(43) Date of publication of application: **19.01.2011**
(21) Application number: 10183233.5
(22) Date of filing: 11.08.2006
(51) Int. Cl.: C12N 1/21, C12P 13/06, C12P 13/08, C12P 13/12, C12P 13/20

(54) **Process for the preparation of aspartate and derived amino acids employing a microorganism with enhanced glyoxylate shunt**

(30) Priority: 11.08.2005 WO PCT/IB2005/003071
(62) Divisional of application: 06792801.0
(71) Applicant: Metabolic Explorer, 63360 Saint Beauzire (FR)
(72) Inventor: FIGGE, Rainer, 63450, LE CREST (FR); BESTEL-CORRE, Gwénaëlle, 63360, SAINT BEAUZIRE (FR); RAYNAUD, Céline, 63111, DALLET (FR); SOUCAILLE, Philippe, 31450, DEYME (FR)
(74) Representative: Tetaz, Franck Claude Edouard

(57) **Abstract**

The present invention relates to increasing the production of aspartate or aspartate-derived metabolites by boosting the activity of the glyoxylate shunt. This is accomplished by increasing the activity of glyoxylate shunt specific enzymes and decreasing the activity of reactions consuming glyoxylate and its precursors.

## Description

### Field of the invention

The present invention relates to increasing the production of aspartate or aspartate-derived metabolites by boosting the activity of the glyoxylate shunt. This is accomplished by increasing the activity of glyoxylate shunt specific enzymes and decreasing the activity of reactions consuming glyoxylate and its precursors.

### Prior art

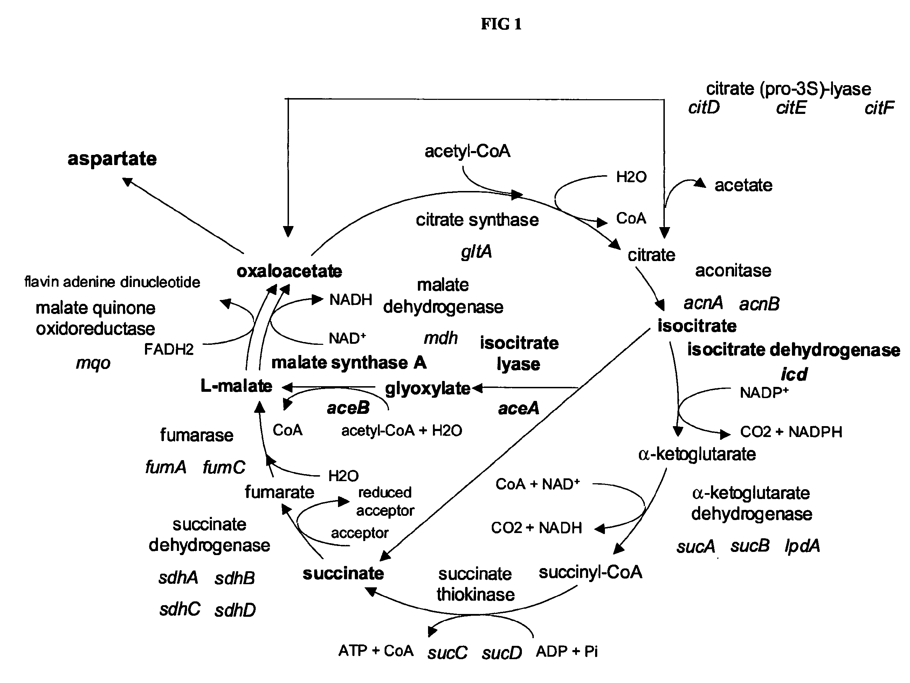

Amino acids and their derivatives are important precursors in the pharmaceutical industry and added to a wide variety of food and feed as supplements. Several amino acids, such as glutamate, lysine and threonine are produced using their natural biosynthetic pathways. In natural amino acid biosynthesis the amino acid aspartate serves as the precursor for the production of other amino acids, such as lysine, threonine, isoleucine and methionine. Aspartate is produced from oxaloacetate, which is a central metabolite of the citric acid cycle. Oxaloacetate can be transformed into a molecule of citrate by accepting a molecule of acetyl-CoA, a reaction that is part of the citrate cycle or it can leave the citrate cycle via a transamination reaction that yields aspartate. In the citrate cycle oxaloacetate fulfils a pure acceptor role and is regenerated during a round of the cycle. Keeping the oxaloacetate pool at a constant level is thus a prerequisite for sufficient citrate cycle activity, which in turn is important for the cellular energy balance. Therefore oxaloacetate that is withdrawn from the cycle needs to be replenished. In *E*. *coli* this is accomplished by several different reactions known as anaplerotic pathways. If *E*. *coli* grows on glucose in a synthetic minimal medium, most of the oxaloacetate required is produced by carboxylation of phosphoenol pyruvate (PEP), a reaction catalyzed by PEP carboxylase (Kay et al., PNAS 1999, 96, 823-28 and references therein). If the carbon source is acetate, oxaloacetate is produced via the glyoxylate cycle (Tricarboxylic acid cycle and glyoxylate bypass, reviewed in Neidhardt, F. C. (Ed. in Chief), R. Curtiss III, J. L. Ingraham, E. C. C. Lin, K. B. Low, B. Magasanik, W. S. Reznikoff, M. Riley, M. Schaechter, and H. E. Umbarger (eds). 1996. Escherichia coli and Salmonella: Cellular and Molecular Biology. American Society for Microbiology). In this cycle isocitrate is cleaved into succinate and glyoxylate, a reaction that is catalyzed by isocitrate lyase, encoded by the *aceA* gene. Succinate directly enters the citric acid cycle and is converted into oxaloacetate. Glyoxylate is converted into malate by incorporating a molecule of acetyl-CoA derived from acetate (see fig. 1), a reaction catalyzed by the enzyme, malate synthase, encoded by *ace*B. Malate also enters the citrate cycle and is converted into oxaloacetate. Thus another oxaloacetate molecule is generated and can leave the citric acid cycle to be transformed into aspartate.

The entry of carbon into the glyoxylate shunt is regulated on the transcriptional and posttranscriptional level. Transcriptional regulation is exerted on the *ace*BAK operon by the IclR repressor. AceBAK encode malate synthase, isocitrate lyase and isocitrate kinase/phosphatase, respectively. The *icl*R gene is negatively autoregulated and activated by the FadR protein. The activity of isocitrate dehydrogenase, encoded by the *icd* gene, is regulated posttranscriptionally. Isocitrate dehydrogenase and isocitrate lyase compete for the common substrate isocitrate. Since the Kₘ value for isocitrate is significantly higher for the isocitrate lyase reaction, the entry into the glyoxylate pathway depends in part on the regulation of the isocitrate dehydrogenase enzyme. Isocitrate dehydrogenase activity is modulated by its phosphorylation or dephosphorylation catalyzed by AceK. Phosphorylation reduces the activity of Icd and dephosphorylation reactivates the Icd enzyme. If AceK acts as kinase or phosphatase depends on the presence of several metabolites. Depletion of isocitrate and 3-phosphoglycerate stimulates kinase activity; the presence of pyruvate and AMP inhibits the kinase function thus favoring the phosphatase activity (see also Neidhard).

A strong production of aspartate is a prerequisite for the industrial production of aspartate-derived amino acids. In *E*. *coli* this has been accomplished by increasing PEP carboxylase activity by expressing feed-back desensitized PEP carboxylase enzymes (Patent EP0723011B1 by Ajinomoto) or heterologous pyruvate carboxylases (patent US6455284). In addition, the deletion of oxaloacetate or malate consuming enzymes, such as pyruvate carboxykinase, encoded by the *pck* gene, or the malic enzymes encoded by *sfc*A and *mae*B, is important for the production of aspartate derived metabolites. An increase of glyoxylate shunt activity has been shown to be beneficial for the production of succinate (Lin, H., Bennett, G. N. San, K-Y. 2005, Biotechnol and Bioeng 89, 148-156). Here we demonstrate that an increase in the activity of the glyoxylate shunt specific enzymes together with a decrease in the consumption of glyoxylate and its precursors can boost the production of aspartate-derived metabolites.

### Summary of the invention

The invention is based on the discovery that an increase in the activity of the glyoxylate shunt can boost the production of aspartate and aspartate-derived metabolites. Thus the invention relates to optimizing a method for the production of aspartate or aspartate-derived metabolites by up-regulating the activity of the glyoxylate shunt and/or decreasing isocitrate dehydrogenase activity. This can be accomplished by directly overexpressing the activity of the enzymes isocitrate lyase and malate synthase, by decreasing their repression through the attenuation, preferentially deletion of the gene *icl*R, or through the deletion of *fad*R. A further increase in the production of aspartate or its derivatives can be achieved by reducing the consumption of glyoxylate and its precursors.

Thus the invention concerns a method for the production of aspartate or its derivatives by culture of a microorganism in an appropriate culture medium and recovery of the aspartate derivative from the culture medium, wherein the microorganism is transformed to enhance the activity of the glyoxylate shunt.

The invention further relates to microorganisms, preferentially enterobacteriaceae, coryneform bacteria or yeast, into which the aforementioned modifications were integrated. Finally the invention describes a method for the fermentative production and purification of aspartate and its derivatives using microorganisms with the described properties, comprising
a) Fermentation of the microorganism producing the desired metabolite
b) Concentration of the desired product in the cells of the bacteria or in the medium and
c) Isolation of the desired metabolite/ constituents of the fermentation broth and/or the biomass optionally remaining in portions or in the total amount (0-100%) in the end product.

### Detailed description of the invention

According to the present invention, "aspartate derived metabolites" or "aspartate derivatives" means any compounds or products synthesized/produced by a microorganism using aspartate as substrate or co-substrate upstream in the biosynthesis pathway. Preferred "aspartate derived metabolites" or "aspartate derivatives" are selected among the amino acids lysine, threonine, isoleucine and methionine, preferably methionine.

Aspartate-derived metabolites, especially the amino acids lysine, threonine and methionine are produced in industrial scale and mainly used in the nutrition of animals and in pharmaceutical applications. Difficult to synthesize chemically as pure L-stereoisomers, some of these amino acids are produced more economically by fermentation. This requires strong production of aspartate and thus a strong efflux of the acceptor molecule oxaloacetate from the citric acid cycle. To counteract the efflux of this acceptor C4 dicarboxylic acid, the oxaloacetate pool needs to be replenished via anaplerotic reactions.

The object of this invention is thus an innovative way to increase the production of oxaloacetate that in tum can leave the citric acid cycle and be transaminated to aspartate. This is accomplished by (i) increasing the activity of the glyoxylate pathway specific enzymes AceA and AceB, (ii) decreasing the activity of isocitrate dehydrogenase that competes with the first enzyme of the glyoxylate shunt, isocitrate lyase, for the substrate isocitrate and (iii) by reducing glyoxylate consuming reactions.

The glyoxylate shunt is an anaplerotic pathway that diverges from the citric acid cycle. In the glyoxylate shunt the common substrate isocitrate is converted into glyoxylate and succinate, a reaction catalyzed by isocitrate lyase encoded by the *ace*A gene. In the second reaction of the glyoxylate cycle glyoxylate is transformed into malate using acetyl-CoA, a reaction catalyzed by malate synthase, encoded by the *ace*B gene. Entry into the glyoxylate shunt is regulated on the transcripdonal level by the transcription factor Ic1R that represses the *ace*BAK operon and by regulating the enzymatic activity of isocitrate dehydrogenase, encoded by the *icd* gene, via phosphorylation. The corresponding kinase/ phosphatase is encoded by the *ace*K gene.

Object of the invention is the increased expression, especially overexpression of the glyoxylate pathway specific enzymes, isocitrate lyase and malate synthase. For this purpose the corresponding genes *ace*A and *ace*B may be encoded chromosomally or extrachromosomally. Chromosomally there may be one or several copies on the genome that can be introduced by methods of recombination known to the expert in the field. Extrachromosomally genes may be carried by different types of plasmids that differ with respect to their origin of replication and thus their copy number in the cell. They may be present as 1-5 copies, ca 20 or up to 500 copies corresponding to low copy number plasmids with tight replication (pSC101, RK2), low copy number plasmids (pACYC, pRSF1010) or high copy number plasmids (pSK bluescript II).

The genes *ace*A and *ace*B may be expressed using promoters with different strength that need or need not to be induced by inducer molecules. Examples are the promoters Ptrc, Ptac, Plac, the lambda promoter cI or other promoters known to the expert in the field.

Expression of the two genes may be boosted or reduced by elements stabilizing or destabilizing the corresponding messenger RNA (Carrier and Keasling (1998) Biotechnol. Prog. 15, 58-64) or the protein (e.g. GST tags, Amersham Biosciences)

The present invention also relates to microorganisms that contain one or several alleles encoding isocitrate dehydrogenase and/or malate synthase according to the invention.

Such strains are characterized by the fact that they possess a carbon metabolism that permits the increased synthesis of oxaloacetate.

In another embodiment of the invention an increase in isocitrate lyase and malate synthase activity is achieved by attenuating the expression of the gene *icl*R encoding a repressor of the *ace*BAK operon. Attenuation is defined as the reduced expression obtained by decreasing the force of the proper promoter or introducing artificial promoters upstream of *icl*R, or possibly as the complete elimination of the *icl*R gene.

In another object of the invention the activity of isocitrate lyase and malate synthase are increased by attenuating, or preferentially eliminating the activation of *icl*R transcription through FadR. This may be accomplished by attenuating, preferentially eliminating the expression of FadR or by deleting the binding sites of FadR in the *icl*R promoter.

In another embodiment of the invention the activity of isocitrate dehydrogenase that competes with isocitrate lyase for substrate is attenuated. This can be accomplished by introducing artificial promoters that drive the expression of the *icd* gene or possibly by introducing mutations into the led enzyme that reduce its activity. Since the activity of Icd is reduced by phosphorylation it may also be controlled by introducing mutant *ace*K genes that have increased kinase activity or reduced phosphatase activity compared to the wildtype AceK enzyme.

In another preferred embodiment of the invention the production of oxaloacetate and thus aspartate and its derived metabolites is further increased by attenuating the expression of genes involved in the consumption of glyoxylate, such as the gene *gcl,* encoding glyoxylate carboligase or *eda* encoding 2-keto 3-deoxygluconate 6-phosphate aldolase (Vlahos & Dekker (1986) JBC 261, pp. 11049-11055).

A further increase of the production of aspartate and aspartate-derived metabolites is obtained by increasing the expression, preferentially by overexpressing other genes that are involved in the production of aspartate or its precursor oxaloacetate. These are the genes by preference *pp*c, encoding phosphoenol pyruvate (PEP) carboxylase and /or pyruvate carboxylase (pyc). In a preferential application of the invention the PEP carboxylase may not be feed-back inhibited by aspartate. The expressed pyruvate carboxylase enzyme may be insensible to feed-back inhibition by aspartate and not require activation by acetyl-phosphate.

To reduce futile cycling between C4 and C3 metabolites the activity of the enzyme phosphoenolpyruvate carboxykinase, encoded by the *pck* gene, may be attenuated, preferentially deleted. Additional mutations that reduce the activity of acetate kinase and phosphotransacetylase encoded by the *pta-ack*A operon may allow the strain to regain vitality. Attenuating the activity of the malic enzyme encoded by sfcA may also reduce futile cycling.

Expressing higher levels of aspC encoding aspartate aminotransferase may also increase aspartate production.

To further increase the production of aspartate the following genes encoding enzymes involved in the transformation of aspartate may be attenuated:
*asp*A encoding aspartase
*pur*C encoding SAICAR synthase
*pur*A encoding adenylosuccinate synthase
*pyr*B, *pyr*L encoding aspartate carbamoyl transferase
*asn*A encoding aspartate ammonia ligase
*asn*B encoding asparagine synthetase B
*arg*G encoding argininosuccinate synthase
*pan*B encoding aspartate decarboxylase
*nad*B aspartate oxidase
*ara*T encoding aromatic-amino-acid: 2-oxoglutarate aminotransferase

Another embodiment of the invention describes the fermentative production of aspartate or one of its derived metabolites, in particular methionine, based on a strain incorporating the mutations described above. In this method the production strain is fermented under industrial conditions that are known to the expert in the field. After the fermentation the desired aspartate derived metabolite can be present inside the cells or in the fermentation broth. The invention comprises also a method for the isolation of the desired metabolite that is known to the expert in the field. In a specific embodiment constituents or biomass from 0 to 100 percent of the fermentation broth may be retained during the preparation of the desired product.

A microorganism that is optimized for the production of aspartate-derived metabolites that harbors the modifications described above is also object of the invention. This microorganism is preferentially yeast, a Corynebacterium or Enterobacterium. Here we have described and exemplified the application for an *Escherichia coli* strain. This, however, does not exclude similar applications in other microorganisms.

### Brief description of the figures

Figure 1 shows the Citrate and Glyoxylate cycle. Shown are the major reactions of the two metabolic pathways including metabolites and genes encoding the relevant enzyme activities.

### Examples:

### 1 Construction of strains with increased activity in the glyoxylate cycle MG1655 metA*11 ΔmetJ ΔfadR (pME101-thrA*1)

To delete the *fad*R gene the homologous recombination strategy described by Datsenko & Wanner (2000) was used. This strategy allows the insertion of a chloramphenicol or a kanamycin resistance cassette, while deleting the gene completely or in part. For this purpose the following oligonucleotides were used:
DfadRF (SEQ ID NO 1)
ggcgcaaagcxcggcgggtttcgcggaagagtacattattgaaagtatctggaataaccgcttccctcccgggactattTGT AGGCTGGAGCTGCTTCG

with
- a region (lower case) homologous to the sequence (1234172-1234251) of the gene *fad*R (reference sequence on the website http://genolist.pasteur.fr/Colibri/),
- a region (upper case) for the amplification of the chloramphenicol resistance cassette (reference sequence in Datsenko, K.A. & Wanner, B.L., 2000, PNAS, 97: 6640-6645),
DfadRR (SEQ ID NO 2))

cccctgaatggctaaatcacccggcagatttttctgcatccggtgccaaatctcgccactctcatgcccatagcgacgC ATATGAATATCCTCCTTAG

with
- a region (lower case) homologous to the sequence (1234874-1234795) of the gene *fad*R (reference sequence on the website http://genolist.pasteur.fr/Colibri/),
- a region (upper case) for the amplification of the chloramphenicol resistance cassette,

The oligonucleotides DfadRF and DfadRR were used to amplify the chloramphenicol resistance cassette from the plasmid pKD3. The PCR product obtained was then introduced by electroporation into the strain MG1655 (pKD46), in which the Red recombinase enzyme expressed permitted the homologous recombination. Chloramphenicol resistant transformants were selected and the insertion of the resistance cassette verified by a PCR analysis with the oligonucleotides fadRF and fadRR defined below. The strain retained was designated MG1655 Δ*fad*R::Cm

fadRF (SEQ ID NO 3) : ccggggagcagcgggtagcatttcagggcc (homologous to the sequence from 12323741 to 1233770).

fadRR (SEQ ID NO 4) : cgccggttccgactggctggaaacgctgc (homologous to the sequence from 1235176 to 1235348).

To boost methionine biosynthesis the *met*J gene encoding the methionine repressor was deleted and feedback resistant mutants of *met*A (*metA*11*) were introduced into the Δ*met*J mutant. These constructions have been described in patent application PCT IB2004/001901. The strain retained was designated MG1655 *metA*11* Δ*met*J.

To transfer the deletion Δ*fad*R::Cm into the strain MG1655 *metA*11* Δ*met*J, the method of phage P1 transduction was used. The protocol followed was implemented in 2 steps with the preparation of the phage lysate of the strain MG1655 Δ*fad*R::Cm and the subsequent transduction into strain MG1655 *metA*11* Δ*met*J.

### Preparation of phage lysate P1:

- Inoculation with 100 µl of an overnight culture of the strain MG1655 Δ*fad*R::Cm of 10 ml of LB + Km 50 µg/ml + glucose 0.2% + CaCl₂ 5 mM.
- Incubation for 30 min at 37°C with shaking.
- Addition of 100 µl of phage lysate P1 prepared on the strain MG1655 (about 1.10⁹ phage/ml)
- Shaking at 37°C for 3 hours until all the cells were lysed.
- Addition of 200 µl chloroform and vortexing.
- Centrifugation for 10 min at 4500 g to eliminate cell debris.
- Transfer of supernatant to a sterile tube and addition of 200 µl chloroform.
- Storage of lysate at 4°C.

### Transduction

- Centrifuging for 10 min at 1500 g of 5 ml of an overnight culture of the strain MG1655 *metA*11* Δ*met*J in LB medium.
- Suspension of the cell pellet in 2.5 ml of 10 mM MgSO₄, 5 mM CaCl₂
- Control tubes: 100 µl cells 100 µl phages P1 of strain MG1655 (Δ*fad*R::Cm)
- Test tube: 100 µl of cells + 100 µl of phages P1 of the strain MG1655 (Δ*fad*R::Cm)
- Incubation for 30 min at 30°C without shaking.
- Addition of 100 µl of 1 M sodium citrate in each tube and vortexing.
- Addition of 1 ml ofLB
- Incubation for 1 hour at 37°C with shaking
- Spreading on dishes LB + Km 50 µg/ml after centrifuging of tubes for 3 min at 7000 rpm.
- Incubation at 37°C overnight.

### Verification of the strain

Chloramphenicol resistant transformants were then selected and the deletion of the gene (Δ*fad*R::Cm) was verified by PCR analysis with the oligonucleotides fadRF and fadRR. The strain retained was designated MG1655 *metA*11* Δ*met*J Δ*fad*R::Cm.

The chloramphenicol resistance cassette can then be eliminated. The plasmid pCP20 carrying FLP recombinase acting at the FRT sites of the chloramphenicol resistance cassette was introduced into the recombinant strain by electroporation. After a series of cultures at 42°C, the loss of the chloramphenicol resistance cassette was verified by PCR analysis with the same oligonucleotides as used previously (fadRF and fadRR). The strain retained is designated MG1655 *metA*11* Δ*met*J Δ*fad*R.

To further boost the production of homoserine the aspartokinase/homoserine a thrA* allele with reduced feed-back resistance to threonine is expressed from the plasmid pCL1920 (Lerner & Inouye, 1990, NAR 18, 15 p 4631) using the promoter Ptrc. For the construction of plasmid pME101-thrA*1 *thr*A was PCR amplified from genomic DNA using the following oligonucleotides:
*Bsp*H1thrA (SEQ ID NO 5): ttaTCATGAgagtgttgaagttcggcggtacatcagtggc
*Sma*1thrA (SEQ ID NO 6): ttaCCCGGGccgccgccccgagcacatcaaacccgacgc

The PCR amplified fragment is cut with the restriction enzymes *Bsp*HI and *Sma*I and cloned into the *Nco*I / *Sma*I sites of the vector pTRC99A (Stratagene). For the expression from a low copy vector the plasmid pME101 is constructed as follows. The plasmid pCL1920 is PCR amplified using the oligonucleotides PME101F and PME101R and the *Bst*Z17I-*Xmn*I fragment from the vector pTRC99A harboring the *lacI* gene and the Ptrc promoter is inserted into the amplified vector. The resulting vector and the vector harboring the *thr*A gene are restricted by *Apa*I and *Sma*I and the *thr*A containing fragment is cloned into the vector pME101. To relieve ThrA from feed-back inhibition the mutation F318S is introduced by site-directed mutagenesis (Stratagene) using the oligonucleotides ThrAF F318S for and ThrAR F318S, resulting in the vector pME101-thrA^{*}1.
PME101F (SEQ ID NO 7): Ccgacagtaagacgggtaagcctg
PME101R (SEQ ID NO 8): Agcttagtaaagccctcgctag
ThrAF F318S (Smal) (SEQ ID NO 9): Ccaatctgaataacatggeaatgtccagcgtttctggcccggg
ThrAR F318S (Smal) (SEQ ID NO 10): Cccgggccagaaacgctggacattgccatgttattcagattgg

The plasmid pME101-*thr*A*1was then introduced into the strain MG1655 *metA*11* Δ*met*J Δ*fad*R::Cm, yielding MG1655 *metA*11* Δ*met*J Δ*fad*R (pME101-*thr*A*1).

### MG1655 metA*11 ΔmetJ ΔiclR (pME101-thrA*1).

The *icl*R gene deletion was introduced in the MG1655 *metA*11* Δ*met*J strain using the strategy described by Datsenko and Wanner (see above) with the following oligonucleotides:
DiclF (SEQ ID NO 11): 
Cgcacccattcccgcgaaacgcggcagaaaacccgccgttgccaccgcaccagcgactggacaggttcagtctttaacgc
gTGTAGGCTGGAGCTGCTTCG
 with
   - a region (lower case) homologous to the sequence (4221202-221120) of the gene *icl*R (reference sequence on the website http://genolist.pasteur.fr/Colibri/),
   - a region (upper case) for the amplification of the kanamycin resistance cassette (reference sequence in Datsenko, K.A. & Wanner, B.L., 2000, PNAS, 97: 6640-6645),
DiclR (SEQ ID NO 12): 
gcgcattccaccgtacgccagcgtcacttccttcgccgctttaatcaccatcgcgccaaactcggtcacgcggtcatcg
gCATATGAATATCCTCCTTAG
 with
   - a region (lower case) homologous to the sequence (4220386-4220465) of the gene *icl*R (reference sequence on the website http://genolist.pasteur.fr/Colibri/),
   - a region (upper case) for the amplification of the kanamycin resistance cassette,

The oligonucleotides DiclF and DiclR were used to amplify the kanamycin resistance cassette from the plasmid pKD4. The PCR product obtained was then introduced by electroporation into the strain MG1655 *met*A*11 Δ*met*J (pKD46). Kanamycin resistant transformants were selected and the insertion of the resistance cassette verified by PCR using the oligonucleotides iclF and iclR defined below. The strain retained is designated MG1655 *met*A*11 Δ*met*J Δ*icl*R::Km

IclF (SEQ ID NO 13) : cctttgaggtcgcatggccagtcggc (homologous to the sequence from 4221558 to 4221533).

iclR (SEQ ID NO 14) : gctttttaatagaggcgtcgccagctccttgcc (homologous to the sequence from 4219917 to 4219949).

The pME101-*thr*A*1 plasmid was then introduced in the strain MG1655 *met*A*11 Δ*met*J Δ*icl*R::Km.

The kanamycin resistance cassette was eliminated using the strategy described above. The loss of the kanamycin resistance cassette was verified by a PCR analysis with the same oligonucleotides as those used previously (iclF and iclR).

The strain retained is designated MG1655 *met*A*11 Δ*met*J Δ*icl*R*.*

### MG1655 metA*11 ΔmetJ ΔfadR ΔiclR (pME101-thrA*1)

The deletion Δ*fad*R::Cm was transferred into the strain MG1655 *met*A*11 Δ*met*J Δ*icl*R::Km by P1 phage transduction as previously described using the phage lysate of the strain MG1655 Δ*fad*R::Cm.

Chloramphenicol and at the same time kanamycin resistant transformants were selected and the deletions Δ*icl*R::Km and Δ*fad*R::Cm verified by PCR with the oligonucleotides fadRF, fadRR and iclF, iclR. The strain retained was designated MG1655 *met*A*11 Δ*met*J Δf*ad*R::Cm Δ*icl*R::Km.

The plasmid pME101-thrA^{*}1 was then introduced in the strain MG1655 *met*A*11 Δ*met*J Δ*fad*R::Cm Δ*icl*R::Km.

The kanamycin and chloramphenicol resistance cassettes were eliminated and their loss verified by PCR analysis with the same oligonucleotides as those used previously (fadF, fadR and iclF, iclR). The strain retained was designated MG1655 *met*A*11 Δ*mef*J Δ*fad*R Δ*icl*R. Subsequently the plasmid pME101-*thr*A*1 was introduced giving the strain MG1655 *met*A*11 Δ*met*J Δ*fad*R Δ*icl*R (pME101-*thr*A*1).

### MG1655 metA*11 ΔmetJ ΔiclR Δgcl (pME101-thrA*1)

The MG1655 Δ*gcl*::Km was constructed using the method of Datsenko and Wanner as described with the following oligonucleotides :
DgclF (SEQ ID NO 15): 
ggcaaaaatgagagccgttgacgcggcaatgtatgtgctggagaaagaaggtatcactaccgccttcggtgttccgggagcT
GTAGGCTGGAGCTGCTTCG
 with
   - a region (lower case) homologous to the sequence (533142-533222) of the region of the gene *gcl* (reference sequence on the website http://genolist.pasteur.fr/Colibri/),
   - a region (upper case) for the amplification of the kanamycin resistance cassette (reference sequence in Datsenko, K.A. & Wanner, B.L., 2000, PNAS, 97: 6640-6645),
DgipR (SEQ ID NO 16) 
gcgttacgttttaacggtacggatccatccagcgtaaaccggcttccgtggtggtttggggtttatattcacacccaacccCATA
TGAATATCCTCCTTAG
with
- a region (lower case) homologous to the sequence (535720-535640) of the region of the gene *gcl* (reference sequence on the website http://genolist.pasteur.fr/Colibri/),
- a region (upper case) for the amplification of the kanamycin resistance cassette,

The oligonucleotides DgclF and DgipR were used to amplify the kanamycin resistance cassette from the plasmid pKD4. The PCR product obtained is then introduced by electroporation into the strain MG1655 (pKD46). Kanamycin resistant transformants were selected and the insertion of the resistance cassette was verified by PCR with the oligonucleotides gclF and gipR defined below. The strain retained was designated MG1655 Δ*gcl*::Km.

gclF (SEQ ID NO 17) : ggatatgcccaccttgctgaagg (homologous to the sequence from 532795 to 532817).

gipR (SEQ ID NO 18) : cgcttagtttcaatcggggaaatgg (homologous to the sequence from 536114 to 536090).

The deletion of Δ*gcl*::Km was transduced into strain MG1655 *met*A*11 Δ*met*J Δ*icl*R using a phage P1 lysate of the strain MG1655 Δ*gcl*::Km

Kanamycin resistant transformants were selected and the deletion of the gene (Δ*gcl*::Km) was verified by PCR with the oligonucleotides gclF and gipR (defined above). The strain retained was designated MG1655 *met*A*11 Δ*met*J Δ*icl*R Δ*gcl*::Km.

The plasmid pNE101-*thr*A*1 was then introduced in the strain MG1655 *met*A*11 Δ*met*J Δ*icl*R Δ*gcl*::Km.

The kanamycin resistance cassette was eliminated as described and its loss verified by PCR with the oligonucleotides gclF and gipR (see above). The strain retained was designated MG1655 *met*A*11 Δ*met*J Δ*icl*R Δ*gcl.*

### MG1655 metA*11 ΔmetJ ΔiclR Δedd-eda (pME101-thrA*1)

The MG1655 Δ*edd-eda*::Km was constructed using the method of Datsenko & Wanner as described with the following oligonucleotides :
DeddF (SEQ ID NO 19) 
CgcgcgagactcgctctgcttatctcgcccggatagaacaagcgaaaacttcgaccgttcatcgttcgcagttggcatgcggT
GTAGGCTGGAGCTGCTTCG
with
   - a region (lower case) homologous to the sequence (1932582-1932499) of the region of the genes *edd-eda* (reference sequence on the website http://genolist.pasteur.fr/Colibri/),
   - a region (upper case) for the amplification of the kanamycin resistance cassette (reference sequence in Datsenko, K.A. & Wanner, B.L., 2000, PNAS, 97: 6640-6645),
DedaR (SEQ ID NO 20) 
gcttagcgccttctacagcttcacgcgccagcttagtaatgcggtcgtaatcgcccgcttccagcgcatctgccggaaccCAT
ATGAATATCCTCCTTAG
with
- a region (lower case) homologous to the sequence (1930144-1930223) of the region of the genes *edd-eda* (reference sequence on the website http://genolist.pasteur.fr/Colibri/),
- a region (upper case) for the amplification of the kanamycin resistance cassette,

The oligonucleotides DeddF and DedaR were used to amplify the kanamycin resistance cassette from the plasmid pKD4. The PCR product obtained was then introduced by electroporation into the strain MG1655 *met*A*11 (pKD46). Kanamycin resistant transformants were then selected and the insertion of the resistance cassette was verified by PCR analysis with the oligonucleotides eddF and edaR defined below. The strain retained was designated MG1655 *met*A*11 Δ*edd-eda*::Km.

eddF (SEQ ID NO 21) : Gggtagactccattactgaggcgtgggcg (homologous to the sequence from 1932996 to 1932968).

edaR (SEQ ID NO 22) : ccacatgataccgggatggtgacg (homologous to the sequence from 1929754 to1929777).

The deletion Δ*edd-eda*::Km was transduced into strain MG1655 *me*tA*11 Δ*mef*J Δ*icl*R using a P1 phage lysate of the strain MG1655 Δ*edd-eda*::Km*.* Kanamycin resistant transformants were selected and the deletion of the gene (Δ*edd-eda*::Km) was verified by PCR analysis with the oligonucleotides eddF and edaR. The strain retained was designated MG1655 *met*A*11 Δ*met*J Δ*icl*R Δ*edd-eda*::Km.

The plasmid pME101-*thr*A*1 was then introduced in the strain MG1655 *met*A*11 Δ*met*J Δ*icl*R Δ*edd-eda*::Km.

The kanamycin resistance cassette was eliminated as described and its loss was verified by PCR analysis with the oligonucleotides eddF and edaR, described above. The strain retained was designated MG1655 *met*A*11 Δ*met*J Δ*icl*R Δ*edd-eda.*

### MG1655 metA*11 ΔmetJ ΔiclR Δedd-eda Δgcl (pME101-thrA*1)

The MG1655 Δ*edd-eda*::Cm was constructed in the strain MG1655 *met*A*11 as described for the kanamycin resistant mutant, only that in this case the vector pKD3 carrying a chloramphenicol resistance was used.

The deletion Δ*edd-eda*::Cm, was then transduced into strain MG1655 *met*A*11 Δ*met*J Δ*icl*R Δ*gcl*::Km by P1 phage transduction using a phage lysate of the strain MG1655 Δ*edd-eda*::Cm.

Chloramphenicol and at the same time kanamycin resistant transformants were selected and the deletions of the genes Δ*edd-eda*::Cm and Δ*gcl*::Km were verified by PCR with the oligonucleotides eddF, edaR and gclf, gipR (defined above). The strain retained was designated MG1655 *met*A*11 Δ*met*J Δ*icl*R Δ*gel*::Km Δ*edd-eda*::Cm.

The plasmid pME101-*thr*A**1* was then introduced in the strain MG1655 *met*A*11 Δ*met*J Δ*icl*R Δ*gcl*::Km Δ*edd-eda*::Cm.

The kanamycin and chloramphenicol resistance cassettes were eliminated as described and their loss was verified by PCR with the oligonucleotides eddF, edaR and gclF, gipR described above. The strain retained was designated MG1655 *met*A*11 Δ*met*J Δ*icl*R Δ*gcl* Δ*edd-eda.*

### 2. Fermentation of strains with increased production of aspartate-derived metabolites

Strains with modifications that increase the production of aspartate derived metabolites were analyzed in Erlenmeyer flask cultures. Two subsequent seed cultures served to inoculate the cultures from which extracellular metabolites were quantified and the total aspartate yield was calculated. Table 1 gives details about the exact cultivation conditions. Luria Bertani (LB) medium (Molecular cloning (2001) Sambrook J. & Russel D. W. (eds) volume 3) supplemented with 2.5 g/l glucose and modified M9 medium (Anderson, 1946, Proc. Natl. Acad. Sci. USA 32:120-128) that was supplemented with 5 g/l MOPS, 16 g/l glucose and 10 mg/l vitamin B12 were used. Antibiotics (Spectinomycin, Kanamycin, Chloramphenicol) were added if necessary at a concentration of 50 mg/l.

**Table1: Cultivation conditions of Erlenmeyer flask cultures used for the determination of aspartate derived metabolites. Abbreviations: CT cultivation time; T temperature.**

| **Culture** | **MEDIUM** | **INOCULATION** | **CULTIVATION CONDITIONS** |
|---|---|---|---|
| **1^{st} SEED** | 5 ml LB | From glycerol stock | CT = 8h ; T = 37°C |
| | | | Agitation = 200rpm |
| **2^{nd} SEED** | 25 ml modified M9 | 2,5 ml of 1^{st} Seed | CT = 8h; T = 37°C |
| | | | Agitation = 200rpm |
| **FINAL CULTURE** | 50 ml modified M9 | Volume of 2^{nd} Seed required to obtain an OD600 of 0,4 | CT = 16h |
| | | | T = 37°C |
| | | | Agitation = 200rpm |

Extracellular metabolites were analyzed in the final culture. Amino acids were quantified by HPLC after OPA/Fmoc derivatization and other relevant metabolites were analyzed using GC-MS after silylation. Glucose concentrations were determined by HPLC analysis using refractive index detection. For the calculation of the aspartate metabolite yield the evaporation during the culture was taken into account.

**Table 2. Sum of major aspartate-derived metabolites (Asp: O-succinylhomoserine, homoserine, threonine, lysine, methionine, isoleucine) produced in batch culture and yield of aspartate derived metabolites with respect to consumed glucose.**

| Genotype | Asp (mmol/g DW) | yield: Asp (mol)/ glucose (mol) % |
|---|---|---|
| MG1655 *met*A*11 Δ*met*J (pME101-*thr*A*1) | 0.86 | 1.87 |
| MG1655 *met*A*11 Δ*met*J Δ*icl*R (pME101-*thr*A*1) | 1.00 | 223 |
| MG1655 *met*A*11 Δ*met*J Δ*icl*R Δ*edd-eda*(pME101-*thr*A***1) | 1.09 | 2.45 |
| MG1655 *met*A*11 Δ*met*J Δ*fad*R (pME101-*thr*A*1) | 127 | 2.80 |
| MG1655 *met*A*11 Δ*met*J Δ*icl*R Δ*fad*R (pME101-*thr*A*1) | 1.02 | 2.36 |

As can be seen from table 2 the amount of aspartate-derived metabolites is significantly increased upon deletion of *icl*R and/or *fad*R thus demonstrating that the deletion of the glyoxylate shunt regulation is crucial for the production of aspartate-derived metabolites. Similarly the yield of aspartate derived metabolites with respect to the consumed glucose is significantly increased. Deletion of *edd-eda* further increases the production of aspartate derived metabolites in the *icl*R deletion mutant.

### 3. Determination of glyoxylate shunt specific enzyme activities

The activities of isocitrate lyase (AceA) and malate synthase (AceB) were determined *in vitro. E. coli* strains were cultured in minimal medium with 5 g/l glucose and harvested at mid log phase. Cells were resuspended in cold potassium phosphate buffer and sonicated on ice (Branson sonifier, 70W). After centrifugation, proteins contained in the supernatants were quantified (Bradford, 1976).

For the determination of isocitrate lyase activity (AceA) five µl extract were assayed in 50 mM imidazole, 100 mM KCl, 1 mM EDTA, 5 mM MgSO4, 20 mM phenylhydrazine, 5 mM isocitrate for 30 minutes at 25°C. Isocitrate lyase activity was determined according to the rate of glyoxylate phenylhydrazine complex formed that absorbs at 324 nm.

Malate synthase activity was determined by assaying 10 µl extract in 50 mM potassium phosphate buffer (pH 6.5), 5 mM MgCl₂, 0.1 mM Acetyl-CoA, 2 mM glyoxylate. Malate synthase activity was determined according to the reduction in absorbance at 232 nm that is caused by the consumption of acetyl-CoA used for the production of malate.

As can be seen from table 3 the deletion of *icl*R increases significantly the activity of AceA and AceB. This can explain the increased production of aspartate-derived metabolites shown in table 2 above. In contrast in the D*fad*R mutant the activity of these two enzymes is not increased indicating that another independent unidentified mechanism must be operative that increases the production of aspartate-derived metabolite.

**Table 3. Isocitrate lyase activity (AceA) and malate synthase activity (AceB) in mUI/mg protein in strains in which the regulatory genes fadR and iclR were deleted.**

| Genotype | AceA | AceB |
|---|---|---|
| MG1655 *met*A*11 Δ*met*J (pME101-*thr*A*1) | 618 | 242 |
| MG1655 *met*A*11 Δ*met*J Δ*icl*R (pME101-*thr*A*1) | 1342 | 733 |
| MG1655 *met*A*11 Δ*met*J Δ*fad*R (pME101-*thr*A*1) | 580 | 257 |
| MG1655 *met*A*11 Δ*met*J Δ*icl*R Δ*fad*R (pME101- *thr*A*1) | 1304 | 675 |

## Claims

1. A method for the production of aspartate or its derivatives by culture of a microorganism in an appropriate culture medium, and recovery of the aspartate derivative from the culture medium,
wherein the microorganism is transformed to enhance the activity of the glyoxylate shunt, by attenuating the expression of at least one gene selectioned among the following :
• *icd* encoding isocitrate dehydrogenase
• *gcl* encoding glyoxylate carboligase
• *eda* encoding 2-keto 3-deoxy gluconate 6-phosphate aldolase
• *fad*R encoding an activator of *icl*R*,* the repressor of the glyoxylate shunt.

2. The method of claim 1, wherein the expression of the gene iclR is attenuated.

3. A method as claimed in one of claims 1 to 2 in which the expression of additional genes involved in the production of aspartate or its derived metabolites is increased.

4. A method as claimed in one of claims 1 to 3 in which the expression of genes decreasing the production of aspartate or its derived metabolites is attenuated.

5. A method for the fermentative preparation of aspartate or a derived metabolite, in particular methionine its precursors or derivatives as claimed in one of claims 1 to 4 comprising :
a) Fermentation of the microorganism producing the desired metabo lite
b) Concentration of the desired product in the cells of the bacteria or in the medium and
c) Isolation of the desired metabolite/ constituents of the fermentation broth and/or the biomass optionally remaining in portions or in the total amount (0-100%) in the end product.

6. A microorganism used for the fermentative production of aspartate or its derived metabolites, in particular methionine, in which the biosynthesis of aspartate or its derivatives is optimized as described in claims 1 to 5.


SEQUENCE LISTING

<110>  Metabolic Explorer
       Figge, Rainer
       Bestel-Corre, Gwenaelle
       Raynaud, Celine
       Soucaille, Philippe

<120>  Method for the production of aspartate and its derivatives

<130>  350153/D23764

<150>  PCT/IB2005/003071
<151>  2005-08-11

<160>  22

<170>  PatentIn version 3.3

<210>  1
<211>  99
<212>  DNA
<213>  Artificial

<220>
<223>  Synthetic oligonucleotide

<400>  1
ggcgcaaagc ccggcgggtt tcgcggaaga gtacattatt gaaagtatct ggaataaccg      60

cttccctccc gggactattt gtaggctgga gctgcttcg                             99


<210>  2
<211>  98
<212>  DNA
<213>  Artificial

<220>
<223>  Synthetic oligonucleotide

<400>  2
cccctgaatg gctaaatcac ccggcagatt tttctgcatc cggtgccaaa tctcgccact      60

ctcatgccca tagcgacgca tatgaatatc ctccttag                              98


<210>  3
<211>  30
<212>  DNA
<213>  Artificial

<220>
<223>  Synthetic oligonucleotide

<400>  3
ccggggagca gcgggtagca tttcagggcc                                       30


<210>  4
<211>  29
<212>  DNA
<213>  Artificial

<220>
<223>  Synthetic oligonucleotide

<400>  4

cgccggttcc gactggctgg aaacgctgc                                    29

<210>  5
<211>  40
<212>  DNA
<213>  Artificial

<220>
<223>  Synthetic oligonucleotide

<400>  5
ttatcatgag agtgttgaag ttcggcggta catcagtggc                         40

<210>  6
<211>  39
<212>  DNA
<213>  Artificial

<220>
<223>  Synthetic oligonucleotide

<400>  6
ttacccgggc cgccgccccg agcacatcaa acccgacgc                          39

<210>  7
<211>  24
<212>  DNA
<213>  Artificial

<220>
<223>  Synthetic oligonucleotide

<400>  7
ccgacagtaa gacgggtaag cctg                                          24

<210>  8
<211>  22
<212>  DNA
<213>  Artificial

<220>
<223>  Synthetic oligonucleotide

<400>  8
agcttagtaa agccctcgct ag                                            22

<210>  9
<211>  43
<212>  DNA
<213>  Artificial

<220>
<223>  Synthetic oligonucleotide

<400>  9
ccaatctgaa taacatggca atgtccagcg tttctggccc ggg                     43

<210>  10
<211>  43
<212>  DNA
<213>  Artificial

<220>

<223>  Synthetic oligonucleotide

<400>  10
cccgggccag aaacgctgga cattgccatg ttattcagat tgg                43


<210>  11
<211>  101
<212>  DNA
<213>  Artificial

<220>
<223>  Synthetic oligonucleotide

<400>  11
cgcacccatt cccgcgaaac gcggcagaaa acccgccgtt gccaccgcac cagcgactgg    60

acaggttcag tctttaacgc gtgtaggctg gagctgcttc g                       101


<210>  12
<211>  100
<212>  DNA
<213>  Artificial

<220>
<223>  Synthetic oligonucleotide

<400>  12
gcgcattcca ccgtacgcca gcgtcacttc cttcgccgct ttaatcacca tcgcgccaaa    60

ctcggtcacg cggtcatcgg catatgaata tcctccttag                         100


<210>  13
<211>  26
<212>  DNA
<213>  Artificial

<220>
<223>  Synthetic oligonucleotide

<400>  13
cctttgaggt cgcatggcca gtcggc                                         26


<210>  14
<211>  33
<212>  DNA
<213>  Artificial

<220>
<223>  Synthetic oligonucleotide

<400>  14
gctttttaat agaggcgtcg ccagctcctt gcc                                 33


<210>  15
<211>  101
<212>  DNA
<213>  Artificial

<220>
<223>  Synthetic oligonucleotide

<400>  15
ggcaaaaatg agagccgttg acgcggcaat gtatgtgctg gagaaagaag gtatcactac    60

cgccttcggt gttccgggag ctgtaggctg gagctgcttc g                    101

<210>  16
<211>  101
<212>  DNA
<213>  Artificial

<220>
<223>  Synthetic oligonucleotide

<400>  16
gcgttacgtt ttaacggtac ggatccatcc agcgtaaacc ggcttccgtg gtggtttggg      60

gtttatattc acacccaacc ccatatgaat atcctcctta g                    101

<210>  17
<211>  23
<212>  DNA
<213>  Artificial

<220>
<223>  Synthetic oligonucleotide

<400>  17
ggatatgccc accttgctga agg                                          23

<210>  18
<211>  25
<212>  DNA
<213>  Artificial

<220>
<223>  Synthetic oligonucleotide

<400>  18
cgcttagttt caatcgggga aatgg                                        25

<210>  19
<211>  102
<212>  DNA
<213>  Artificial

<220>
<223>  Synthetic oligonucleotide

<400>  19
cgcgcgagac tcgctctgct tatctcgccc ggatagaaca agcgaaaact tcgaccgttc      60

atcgttcgca gttggcatgc ggtgtaggct ggagctgctt cg                   102

<210>  20
<211>  100
<212>  DNA
<213>  Artificial

<220>
<223>  Synthetic oligonucleotide

<400>  20
gcttagcgcc ttctacagct tcacgcgcca gcttagtaat gcggtcgtaa tcgcccgctt      60

ccagcgcatc tgccggaacc catatgaata tcctccttag                      100

<210>  21
<211>  29
<212>  DNA
<213>  Artificial

<220>
<223>  Synthetic oligonucleotide

<400>  21
gggtagactc cattactgag gcgtgggcg                                          29


<210>  22
<211>  24
<212>  DNA
<213>  Artificial

<220>
<223>  synthetic oligonucleotide

<400>  22
ccacatgata ccgggatggt gacg                                               24